# EUROPÄISCHE PATENTSCHRIFT

(11) **EP 3 072 539 B1**
(45) Veröffentlichungstag und Bekanntmachung des Hinweises auf die Patenterteilung: **02.08.2017**
(21) Anmeldenummer: 16158658.1
(22) Anmeldetag: 04.03.2016
(51) Int. Cl.: A61L 31/02, A61L 31/14, A61B 17/86

(54) **RESORBIERBARE METALLISCHE SCHRAUBE MIT ERHÖHTER TORSIONSFESTIGKEIT FÜR DIE OSTEOPATHIE**
RESORBABLE METAL SCREW WITH INCREASED TORSIONAL STRENGTH FOR OSTEOPATHY
VIS METALLIQUE RESORBABLE PRESENTANT UNE TORSION ELEVEE POUR L'OSTEOPATHIE

(30) Priorität: 24.03.2015 US 201562137241 P
(43) Veröffentlichungstag der Anmeldung: 28.09.2016
(73) Patentinhaber: Biotronik AG, 8180 Bülach (CH)
(72) Erfinder: Bayer, Ullrich, 18209 Bad Doberan (DE); Anopuo, Okechukwu, 18119 Rostock (DE); Lootz, Daniel, 18119 Rostock (DE)
(74) Vertreter: Galander, Marcus

(56) Entgegenhaltungen:
- WO-A1-94/07425
- US-A1- 2014 243 911

## Beschreibung

Die Erfindung betrifft eine Knochenschraube mit selbstschneidendem Gewinde sowie ein orthopädisches Implantat sowie schließlich ein Verfahren zur Herstellung solcher Knochenschrauben bzw. Implantate.

Knochenschrauben mit selbstschneidendem Gewinde sind seit Jahrzehnten bekannt und stellen wesentliche Befestigungselemente in der Prothetik dar. Sie sind während der bereits langen Zeitdauer ihres klinischen Einsatzes Gegenstand vielfältiger Verbesserungen sowohl hinsichtlich des Materials als auch hinsichtlich der geometrischen Konfiguration gewesen. Den breitesten Einsatz haben in der Vergangenheit Edelstahl- und Titanschrauben gefunden. Es hat jedoch immer wieder Bestrebungen gegeben, resorbierbare Knochenschrauben und ähnliche orthopädische Implantate mit vergleichbaren Leistungsparametern bereitzustellen. In diesem Zusammenhang sind Knochenschrauben aus Magnesiumlegierungen bekannt geworden; siehe etwa US 2011/0313527 A1, und auch Schrauben auf Mg-Basis mit speziellen Beschichtungen, wie in US 2012/0150295 A1 beschrieben. Die Druckschrift US 2014/243911 A offenbart eine Knochenschraube aus einer Magnesiumlegierung, die zur Erhöhung der Bruchfestigkeit einen verdickten Schraubenkopf aufweist.

Magnesium besitzt im Vergleich zu Titan und Edelstählen geringere Festigkeiten. Dies macht den Einsatz dieser Werkstoffgruppe für selbstschneidende resorbierbare Knochenschrauben die mit einem hohen Drehmoment in das Knochengewebe eingebracht werden müssen problematisch. Besonders bei kleinformatigen und/oder kanülierten Schrauben besteht die Gefahr, dass das anzulegende Drehmoment die Torsionsfestigkeit des Werkstoffs übersteigt und somit die Schraube überdreht wird und bricht.

Der Erfindung liegt daher die Aufgabe zugrunde, eine Knochenschraube bzw. ein entsprechendes orthopädisches Implantat mit verringerter Bruchgefahr und der sich hieraus ergebenden Option einer weiteren Miniaturisierung bereitzustellen. Des Weiteren soll ein Verfahren zur Herstellung derartiger Schrauben bzw. Implantate bereitgestellt werden.

Diese Aufgabe wird in ihrem Vorrichtungsaspekt durch eine Knochenschraube mit den Merkmalen des Anspruchs 1 bzw. ein orthopädisches Implantat mit den Merkmalen des Anspruchs 8 und in ihrem Verfahrensaspekt durch ein Verfahren mit den Merkmalen des Anspruchs 9 gelöst. Zweckmäßige Fortbildungen des Erfindungsgedankens sind Gegenstand der abhängigen Ansprüche.

Die Erfindung schließt die Überlegung ein, die effektive Krafteinwirkung, die aus dem beim Eindrehen der Schraube aufgewandten Drehmoment resultiert, unter die Bruchgrenze des Materials zu drücken. Da aus dem (äußeren) Drehmoment eine (innere) Torsionsspannung resultiert, deren Richtung sich aus der Eindrehrichtung der Schraube (normalerweise im Uhrzeigersinn) ergibt, gehört zur Erfindung weiterhin der Gedanke der Bereitstellung einer entgegengerichteten Torsionsspannung. Diese wird der Knochenschraube erfindungsgemäß im Herstellungsprozess eingeprägt.

Die damit einhergehende geringere Torsionsbelastung der Schraube ermöglicht eine Querschnitts- und damit eine Massereduzierung. Dies leistet einerseits einen wesentlichen Beitrag zur Minimierung der Degradationszeit und reduziert andererseits die Menge an frei werdendem Wasserstoff. Damit wird schließlich eine größere Körperverträglichkeit und eine Reduzierung der Rehabilitationszeit der Patienten erreicht. Außerdem kann eine weitere Miniaturisierung der Schrauben erreicht werden, und es können somit neue Einsatzgebiete resorbierbarer Schrauben im Schädel- und Kleingliedmaßenbereich erschlossen werden.

In einer Ausführung der Erfindung hat die Knochenschraube einen kanülierten Grundkörper, insbesondere mit einem Außendurchmesser im Bereich zwischen 1,5 und 5 mm, spezieller zwischen 2,5 und 3,5 mm, und einem Innendurchmesser im Bereich zwischen 0,5 und 2,5 mm, spezieller zwischen 0,8 und 1,3 mm. Auch hierdurch wird eine Massenreduzierung der Schraube erreicht. Diese Reduktion der Menge an Magnesium führt wiederum zu einer verringerten Degradationszeit und gleichzeitig zu einer Verringerung der Menge an Wasserstoff, der bei der Degradation freigesetzt wird. Dies wiederum verringert die Gefahr der taschenförmigen Einlagerung von Wasserstoff, der vom Körper nicht schnell genug resorbiert werden kann.

In einer weiteren Ausführung der Erfindung weist das selbstschneidende Gewinde ein Sägefeingewindeprofil auf. Grundsätzlich sind auch andere Profilquerschnitte, insbesondere solche mit im Querschnitt gekrümmten Gewindeflanken, möglich. Deren Herstellung ist jedoch möglicherweise aufwändiger als die eines herkömmlichen Sägefeingewindeprofils mit geradlinig verlaufenden Gewindeflanken und daher aus heutiger Sicht nicht bevorzugt.

In einer weiteren Ausgestaltung schließt die Erfindung die Überlegung ein, durch eine Oberflächenfunktionalisierung des selbstschneidenden Gewindeprofils die Reibung beim Eindrehen der Schrauben zu minimieren. Dadurch wird das notwendige Dreh- bzw. Torsionsmoment verringert und die Gefahr des Schraubenbruchs weiter reduziert.

Zugleich ermöglicht diese Oberflächenfunktionalisierung in Kombination mit der Einprägung einer gerichteten Torsionsspannung eine mehrparametrige Optimierung der Knochenschraube unter Beachtung spezifischer Anwendungserfordernisse, einschließlich der jeweiligen Kostensituation. Sofern beispielsweise der zusätzliche Aufwand der Oberflächenbeschichtung kostenseitig Problemlos tragbar ist, kann entweder die resultierende Torsionseigenspannung der Knochenschraube nach Abschluss aller Bearbeitungsschritte geringer gewählt oder deren Masse weitest möglich reduziert werden, um Verträglichkeitsanforderungen bestmöglich zu erfüllen. Sind andererseits die zusätzlichen Verfahrensschritte für eine Oberflächenbeschichtung kostenseitig nicht tragbar, muss auf eine ausreichende Wandstärke und hohe Torsionseigenspannung besonderer Wert gelegt werden.

Das oberflächenfunktionalisierte, selbstschneidende und selbstschmierende Gewindeprofil soll gemäß einer weiteren Überlegung der Erfinder zudem noch so gestaltet sein, dass während und unmittelbar nach dem Schneidvorgang die Knochenheilung beschleunigende Substanzen (wie z. B. eine Proliferation humaner Knochenzellen begünstigende bmp's) freigesetzt werden. Das Gewinde weist dadurch eine multifunktionale Oberfläche auf. Gemäß einem weiteren Aspekt dieser Ausgestaltung wird zusätzlich oder alternativ zur erwähnten Minimierung der Reibung beim Eindrehen der Schraube eine Erhöhung der Selbstschneidwirkung durch einen entsprechend lokalisierten Zusatzstoff vorgeschlagen. Dieser liegt erfindungsgemäß in Form von Mikro-Schleifkörpern in der bioaktiven Oberflächenbeschichtung vor.

Mit anderen Worten: Die Schneidhaltigkeit soll einerseits durch ebenfalls degradierbare Verbindungen erreicht werden (stofflicher Aspekt), anderseits soll durch die zum Einsatz kommenden Schleifkörper die Oberflächenrauheit gezielt erhöht werden (oberflächentopographischer Aspekt). Durch die dadurch entstehende Vergrößerung der realen Oberfläche werden Mikrokavitäten erzeugt, die eine bessere Verankerung von Mikropartikeln ermöglichen. Diese bestehen insbesondere aus polymeren Hohlkugeln, die mit das Knochenwachstum fördernden Substanzen gefüllt sind.

Beim Schneiden bzw. Eindrehen der Schraube in die Knochenspongiosa entstehen lokale Reibmomente an den Gewindespitzen und den Gewindeflanken. Diese führen einerseits zu einer Fragmentierung des harten und spröden Schneidstoffs, andererseits zu kurzzeitig wirkenden, lokal begrenzten Temperaturerhöhungen. Beide Effekte führen zu einer gewollten Zerstörung des polymeren Hüllenmaterials, in deren Ergebnis die das Knochenwachstum stimulierende Wirkstoffe in flüssiger oder pastöser Form freigesetzt werden. Durch die verbesserte Schneidwirkung wird die Reibung während des Einschraubvorgangs minimiert, was zu einer Steigerung der weiter oben erwähnten vorteilhaften Wirkungen der eingeprägten Torsionseigenspannung führt.

In einer weiteren Ausführung sind die Mikro-Schleifkörper der Oberflächenbeschichtung durch Druck und Reibungswärme beim Eindrehen der Knochenschraube in Knochensubstanz zerstörbar, insbesondere in kleinere Partikel umwandelbar, die eine Vergrößerung der effektiven Schraubenoberfläche bewirken. Dies erhöht die wirksame Schneidkraft der Schraube gegenüber dem Knochengewebe und somit letztlich die Selbstschneidwirkung. Auch hieraus ergibt sich wiederum die Möglichkeit einer Reduzierung der Schraubendimensionen, mit den oben erwähnten Vorteilen. Die sich aus den größeren Schleifkörpern bildenden kleinen Partikel bewirken zudem ein schnelleres Einwachsen der Schraube in die Knochenmatrix.

In einer weiteren Ausführung der Erfindung weisen die Mikro-Schleifkörper kristallines Hydroxylapatit auf und sind insbesondere nadelförmig ausgebildet. Die typische Form dieses kommerziell verfügbaren Schleifmittels eignet sich, insbesondere bei entsprechend orientierter Einbindung der Schleifkörper in die Oberflächenbeschichtung, besonders zur Steigerung der Schneidfähigkeit und Schneidhaltigkeit der vorgeschlagenen Knochenschraube.

Unter herstellungstechnischen Aspekten ist es bevorzugt, die gerichtete Torsionseigenspannung der noch unfertigen Knochenschraube, also gewissermaßen einem Halbzeug, einzuprägen. Das Verfahren umfasst die Schritte des Bereitstellens eines Grundkörpers aus einer Magnesiumlegierung und Einformens des selbstschneidenden Gewindes in die Au-ßenwandung des Grundkörpers zur Herstellung der Knochenschraube oder des Knochenschraubenabschnitts des orthopädischen Implantats, wobei im Schritt des Bereitstellens des Grundkörpers oder im Schritt des Einformens mindestens der Grundkontur des selbstschneidenden Gewindes durch eine drehende Führung eines Halbzeugs oder des Grundkörpers in einem Formwerkzeug die der Eindrehrichtung des Gewindes entgegengerichtete Torsionseigenspannung eingeprägt wird.

In einer aus derzeitiger Sicht bevorzugten Verfahrensführung wird der Grundkörper als Rohr oder einen Rohrabschnitt aufweisendes Element bereitgestellt, wobei wiederum bevorzugt das Bereitstellen des Rohres mit gleichzeitigem Einformen des selbstschneidenden Gewindes in einem rotierenden Strangpressen, optional mit nachfolgendem Fräsen oder Schleifen oder Gewindeschneiden, erfolgt.

Bei dieser Verfahrensführung bleibt die eingeprägte gerichtete Torsionseigenspannung - anders als bei einer alternativen möglichen Verfahrensführung, in der zunächst ein einfaches Rohr mit der Torsionseigenspannung versehen und in dieses Rohr später das Gewinde eingearbeitet wird - weitestgehend im Endprodukt erhalten. Tendenziell bleibt sie umso besser erhalten, je geringer die mechanische Einwirkung auf die unfertige Knochenschraube im Schritt der endgültigen Ausformung der Gewindekontur gehalten werden kann. Unter diesem Aspekt ist es aus derzeitiger Sicht bevorzugt, wenn der Materialabtrag beim nachfolgenden Fräsen oder Schleifen oder Gewindeschneiden weniger als 0,2 mm, insbesondere weniger als 0,1 mm, beträgt.

In aus derzeitiger Sicht bevorzugten Parameterkonstellationen wird das rotierende Strangpressen als Warmumformen mit einer Verformungstemperatur im Bereich zwischen 100 °C und 450 °C und/oder mit einer Anzahl von 0,2 bis 2 Umdrehungen pro cm Schraubenlänge bei einem zwischen 2,0 und 3,5 mm liegenden Außendurchmesser ausgeführt. Wie weiter oben erwähnt, sind erfindungsgemäß auch Knochenschrauben mit anderen Außendurchmessern realisierbar; für diese kann aber eine andere Anzahl von Umdrehungen pro cm Schraubenlänge festzulegen sein. Aus derzeitiger Sicht ist weiterhin bevorzugt eine Verfahrensführung, bei der die Dehnrate zwischen 0,05 s⁻¹ und 25 s⁻¹, insbesondere zwischen 0,07 s⁻¹ und 22 s⁻¹, liegt.

Gemäß der weiter oben angesprochenen produktseitigen Ausgestaltung mit einer bioaktiven Oberflächenbeschichtung, die durch Druck und Reibungswärme beim Eindrehen der Knochenschraube in Knochensubstanz zerstörbare Mikrokapseln mit einem Schmierstoff und/oder Mikro-Schleifkörper enthält, ist in einer Ausgestaltung auch eine entsprechende Verfahrensführung vorgesehen. Diese weist die Schritte des Ausbildens einer Beschichtungs-Basis auf mindestens einem Abschnitt der Oberfläche des Gewindes, und Auf-/Einbringens der Mikrokapseln und/oder der Mikro-Schleifkörper auf und/oder in die Beschichtungs-Basis auf.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A und 1B: Darstellungen einer erfindungsgemäßen Knochenschraube,
- Fig. 2: eine Detailansicht aus Fig. 1A,
- Fig. 3A und 3B: Detailansichten aus Fig. 2 zu Ausführungsformen der Erfindung,
- Fig. 4A bis 4B: eine schematische Draufsicht bzw. Seitenansicht eines Halbzeugs zur Herstellung einer erfindungsgemäßen Knochenschraube und
- Fig. 5A bis 5C: schematische perspektivische Darstellungen zur Erläuterung des Formvorganges in einer Ausführungsform des Herstellungsverfahrens.

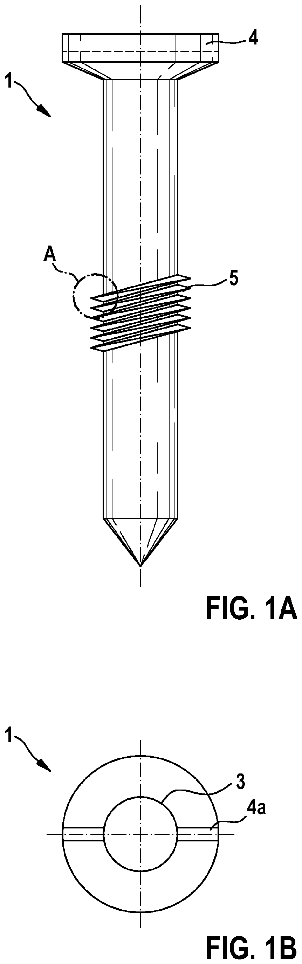

Fig. 1A zeigt in einer skizzenartigen Darstellung (Seitenansicht) eine Knochenschraube 1 aus einer Mg-Legierung, die innen hohl bzw. kanüliert gefertigt ist, also - wie in der Draufsicht auf den Schraubenkopf gemäß Fig. 1B zu erkennen ist - mit einem zylindrischen Hohlprofil 3 und die einen Schraubenkopf 4 mit einem Schlitz 4a hat.

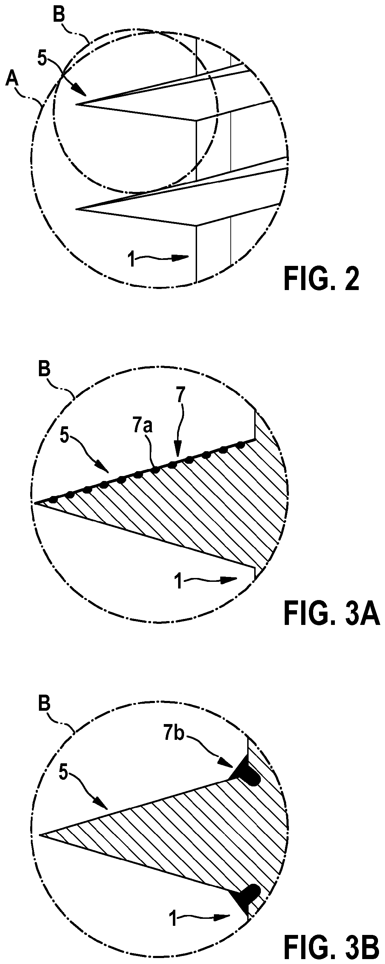

Fig. 2 zeigt in einer schematischen Seitenansicht das Detail A des Schraubengewindes 5 der Knochenschraube 1. Es ist zu erkennen, dass es sich hierbei um ein sehr spitzwinkliges, "scharfes" Sägefeingewindeprofil handelt. Bei der gezeigten Ausführung beträgt der Winkel zwischen den Gewindeflanken ca. 20°, er kann aber in Abhängigkeit vom konkreten Material und Einsatzzweck der Schraube auch einen anderen Wert, insbesondere zwischen 15° und 30°, haben.

Fig. 3A zeigt in einer weiter vergrößerten Ansicht das Detail B aus Fig. 2 in einer Schnittansicht, und zwar speziell eine in Hohlräumen der Gewindeflanke des Schraubengewindes 5 "verankerte" Oberflächenbeschichtung 7 mit eingelagerten Mikro-Schleifkörpern 7a. In der weiteren Detailansicht der Fig. 3B ist (unter Fortlassung der Oberflächenbeschichtung 7) speziell nur gezeigt, dass in einer Ausführung der Erfindung an den Übergängen des Grundkörpers der Knochenschraube 1 zum Gewindeprofil 5 Mikro-Reservoire (Mikrokapseln) 7b eingelagert sind, die mit einem an das Schraubenmaterial einerseits und die Knochensubstanz andererseits angepassten Schmierstoff und gegebenenfalls auch einem medizinischen Wirkstoff befüllt sind. Diese Mikrokapseln 7b können mit den in Fig. 3A gezeigten Mikro-Schleifkörpern 7a kombiniert sein.

Die Mikrokapseln 7b adhärieren sowohl an der Kavitäten aufweisenden, mit mikrokristallinem Hydroxylapatit bedeckten Oberfläche des Gewindeprofils 5 als auch stoff- und formschlüssig in der dünnen, aus einem biodegradierbaren Polymer bestehenden Oberflächenschicht 7, die als Wirtsmatrix für die Mikro-Schleifkörper 7a, etwa aus Hydroxylapatit, fungiert. Es entsteht ein Oberflächenverbund aus harten und schneidfähigen Hydroxylapatitkristallen und dazwischen eingelagerten Mikrokapseln, die wiederum aus einem Hüllenmaterial aus biodegradabierbaren Polymer und darin enthaltenen Wirkstoffen für die Osteosynthese bestehen.

Die Wirkstoffe enthaltenden Mikrokapseln werden beim Schneidvorgang durch die eintretende Fragmentierung der harten, aber auch spröden Hydroxylapatitkörper mechanisch und lokal auch durch thermische Effekte (Mikroreibungswärme) zerstört. Die heraustretende hochviskose wirkstoffbeladene Trägerflüssigkeit ruft einen Selbstschmiereffekt hervor, infolge dessen das aufzubringende Eindrehmoment der Schraube noch weiter reduziert wird. Durch die nun vorliegende wesentliche Reduzierung des Eindrehmoments ergibt sich eine geringere Torsionsbelastung der Schraube. Diese führt zu der konstruktiven Möglichkeit, die Wandstärke der kanülierten Schraube und damit deren Masse im Bereich zwischen 20 und 50 % zu reduzieren. Dadurch wird einerseits eine Festigkeitsreserve geschaffen, die eine weitere Miniaturisierung von Knochenschrauben ermöglicht, ohne die Gefahr des Abdrehens der Schraube durch zu hohe Torsionsbelastung zu erhöhen. Andererseits wird die Degradationsdauer verkürzt und die Menge an freigegebenen Wasserstoff reduziert.

Hydroxylapatite gehören zur Gruppe der Calciumphosphate, die als Knochenersatzwerkstoffe keine körpereigenen Abwehrreaktionen hervorrufen. Hydroxylapatite sind jedoch spröde. Dies drückt sich durch geringe Bruchzähigkeitskennwerte (K_{IC} = 1MPam1/2) aus. Aus diesem Grunde finden Hydroxylapatite in der Regel keine Anwendung für lasttragende orthopädische Implantate, da diese zur Rissbildung neigen und dadurch mechanisch nicht mehr belastbar wären.

Die vorliegende Ausführungsform nutzt diesen vermeintlichen Nachteil ganz gezielt aus. Die in der Magnesiummatrix der Schraube eingelagerten Hydroxylapatitkristalle (Partikelgrößenbereich z.B. 1-5 µm) werden beim Einschraubvorgang komplexen mechanischen Beanspruchungen ausgesetzt. Diese sind gekennzeichnet durch Zug- und Druckspannungen, die teilweise durch Torsionsspannungen überlagert werden. Diese komplexen Spannungen sind bei weitem größer als die Festigkeit der Hydroxylapatitkristalle, und es kommt zur Fragmentierung jener Kristalle. Es entstehen mikrokristalline Partikel (0,1 -1 µm) mit viel größeren realen Oberflächen, die zu einem Teil weiterhin in der Magnesiumoberfläche der Schraube verankert bleiben, zum anderen Teil jedoch von der Oberfläche weg und - unterstützt durch die in den Mikrokapseln enthaltende Flüssigkeit - in die umgebende Knochenspongiosa transportiert werden.

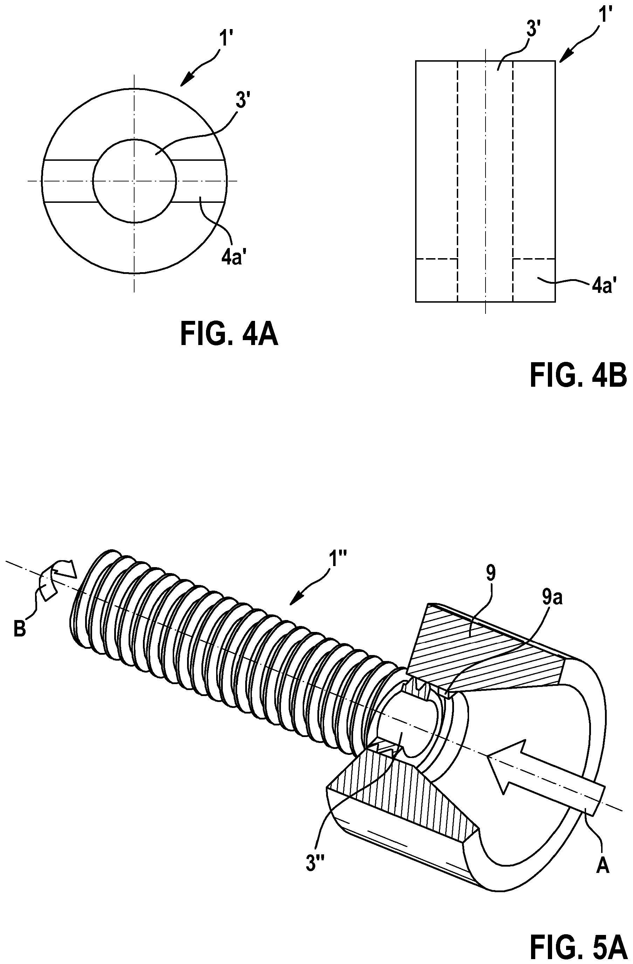

Fig. 4A und 4B zeigen schematisch den Rohling 1' einer erfindungsgemäßen Knochenschraube, der eine axial durchgehende Kanüle 3' und an einer Stirnseite einen quer verlaufenden Schlitz 4a' hat. Dieser Rohling 1' wird durch einen nachfolgenden Strangpress- bzw. Extrudiervorgang (siehe weiter unten) mit einer eingeprägten Torsionseigenspannung versehen. Der Schlitz 4a' dient dabei als Eingriff für den Mitnehmer und ermöglicht das Strangpressen unter Drehen wie nachfolgend geschildert.

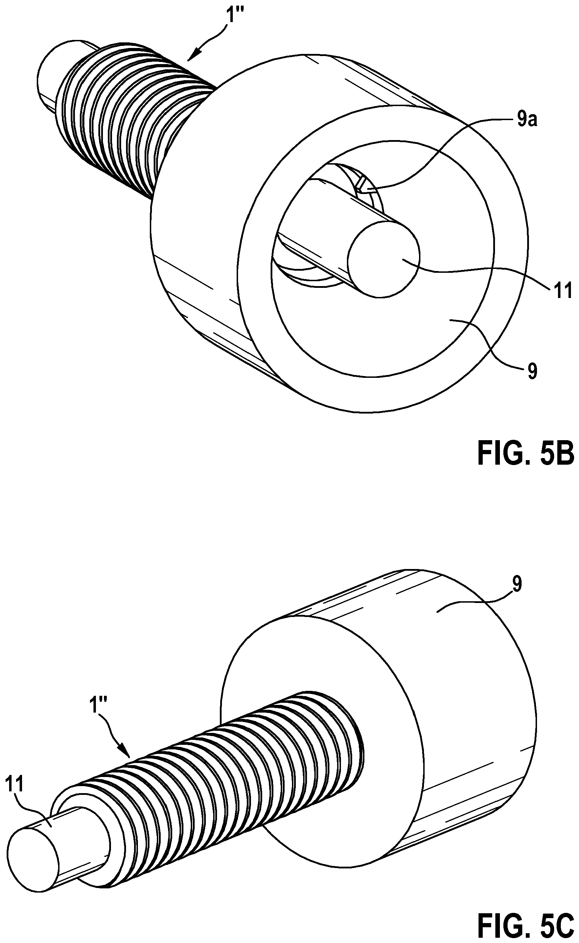

Fig. 5A bis 5C zeigen in schematischen perspektivischen Darstellungen zur Erläuterung eines entsprechenden Strangpress- bzw. Extrudierschrittes ein Knochenschrauben-Vorprodukt 1 " zusammen mit einem Press- bzw. Extrudierkopf 9, durch den das Vorprodukt in eine mit dem Pfeil A bezeichnete Vorschubrichtung unter Drehen in eine mit dem Pfeil B bezeichnete Drehrichtung geführt wurde bzw. in dem es durch Strangpressen oder Extrusion geformt wurde. Ziffer 9a bezeichnet hierbei ein Formgebungselement bzw. eine Innenkontur für die Gewindeextrusion, und Ziffer 11 bezeichnet einen Press- bzw. Extrusionsstempel zur Erzeugung der zentrischen Kanüle 3" des Knochenschrauben-Vorproduktes 1" (der Mitnehmer des Press- bzw. Extrusionsstempels 11 und der korrespondierende Schlitz 4a' sind in den Figuren 5A bis 5C zur Vereinfachung nicht dargestellt).

Nachfolgend werden beispielhaft Schritte und Verfahrensdetails einer möglichen Verfahrensführung zur Herstellung einer Ausführungsform der erfindungsgemäßen Knochenschraube angegeben:
1. Es wird ein Vorprodukt (Rohling) gemäß Fig. 4A und 4B bereitgestellt, der 12 mm lang, an einer Stirnfläche ca. 4 mm tief geschlitzt ist.
2. Es wird ein Stempel in zylindrisch abgestufter Grundform bereitgestellt, der am Übergang zwischen 1,64 mm Durchmesser und 4,00 mm Durchmesser einen "Mitnehmer" hat.
3. Der Mitnehmer greift in den Rohling ein, und während der Extrusion wird der Rohling drehend in die Passform bzw. Matrize eingeführt; die Drehrichtung ist derjenigen des späteren Gewindes und der Gewindebelastung entgegen gerichtet.
4. Der Verformungsvorgang unter Drehen mit konstanter Winkelgeschwindigkeit wird bis zum Vorliegen eines Knochenschrauben-Vorprodukts der gewünschten Länge fortgeführt.
5. Die Anzahl der Umdrehungen beim Warmumformen in Stangen- bzw. Rohraußendurchmesserbereich zwischen 2,0 und 3,5 mm liegt zwischen 1,0 und 8 pro cm Länge.
6. Die Verformungstemperatur liegt je nach Legierung zwischen 100 °C und 450 °C.
7. Die Dehnraten beim Umformprozess liegen zwischen 0.07 s⁻¹ und 22 s⁻¹.
8. Die Matrize weist eine wendelförmige Innenstruktur auf (ähnlich der Negativform eines Schneckengetriebes).
9. Die Außenoberfläche wird anschließend mit geringem Masseabtrag "scharf" geschliffen, und es entsteht das endgültige stelbstschneidende Gewinde.

In einer Abwandlung dieser Ausführung kann ein extrudiertes Rohr aus der biodegradierbaren Mg-Legierung WE43 oder MgCa0,8 oder aus der biodegradierbaren Mg-Legierung AZ31 eingesetzt werden. Außen- und Innendurchmesser der Schraube und somit die resultierende Wanddicke können ebenso variiert werden wie Gewindetiefe und -steigung und andere Parameter der Schraubenkonfiguration.

Nach dem Formvorgang mit Einprägung der Torsionseigenspannung wird ein selbstschneidendes Sägefeingewindeprofil mit beispielsweise einer Gewindetiefe von 0,5 bis 0,7 mm bei einer beispielhaften Wanddicke des extrudierten Rohres von 1,0 mm mittels eines Schneideisens erzeugt.

In einer weitergehenden Modifikation der vorstehend erläuterten Ausführung wird kein innenkanüliertes Rohr, sondern eine massive Stange als Knochenschrauben-Vorprodukt mittels Strangpressen hergestellt, wobei auch hier eine Außenkontur wie bei der vorgenannten Ausführung eingeformt und eine Torsionseigenspannung eingeprägt wird. Es können beispielsweise massive Knochenschrauben mit einer Länge zwischen 15mm und 40mm und einem Durchmesser zwischen 2mm und 3,5mm erzeugt werden.

Eine beispielhafte Ausführung der weiter oben erwähnten Nachbehandlung zur Erzeugung einer Oberflächenfunktionalisierung sieht wie folgt aus:
1. Es erfolgt eine Reinigung in Isopropanol (Verweilzeit 2 min).
2. Es erfolgt ein Tauchen der Schraube (dip coating) in eine Flüssigkeit aus PLA oder PLA Blends, die Hydroxylapatitpartikel (Partikelgröße zwischen 1 und 10 µm) enthält, bei mindestens 150 °C.
3. Es erfolgt ein Trocknen in einem Umluftofen bei Temperaturen zwischen 60 und 80°C.
4. Es erfolgt ein kurzes Eintauchen (<10 s) der beschichteten Schraube in Chloroform; dadurch wird ein Teil der Oberfläche angelöst. Es entsteht eine selbstklebende, mit Mikrokavitäten versehene mikrorauhe Oberfläche.
5. Danach erfolgt ein sofortiges Herausnehmen und Eintauchen (10-30 s) in eine wässrige Mikrokapseln enthaltende Flüssigkeit. Diese Mikrokapseln bestehen aus einer Hülle aus einem PLA oder PLA Blend. Der Durchmesser dieser Mikrokapseln beträgt zwischen 1 und 10 µm. Diese Mikrokapseln enthalten in ihrem Inneren knochenmorphogenetische Proteine.
6. Es erfolgt ein Herausnehmen und Trocknen in einem Umluftofen bei Temperaturen zwischen 30 und 40°C. Ein Teil der Mikrokapseln hat sich in die Mikrokavitäten der PLA/Hydroxylapatit enthaltenden Oberfläche eingelagert (Formschluss) und/oder klebt an dieser Oberfläche fest (Stoffschluss).

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Knochenschraube (1) mit selbstschneidendem Gewinde (5), im Wesentlichen bestehend aus einer Magnesiumlegierung und versehen mit einer eingeprägten Torsionseigenspannung, die der Eindrehrichtung des Gewindes entgegengesetzt ist.

2. Knochenschraube nach Anspruch 1, wobei die eingeprägte Torsionseigenspannung über die Schraubenlänge konstanten Betrag hat.

3. Knochenschraube nach einem der vorangehenden Ansprüche, welche einen kanülierten (3) Grundkörper, insbesondere mit einem Außendurchmesser im Bereich zwischen 1,5 und 5 mm, spezieller zwischen 2,5 und 3,5 mm, und einem Innendurchmesser im Bereich zwischen 0,5 und 2,5 mm, spezieller zwischen 0,8 und 1,3 mm, aufweist.

4. Knochenschraube nach einem der vorangehenden Ansprüche, wobei das selbstschneidende Gewinde (5) ein Sägefeingewindeprofil aufweist.

5. Knochenschraube nach einem der vorangehenden Ansprüche, welche mindestens in Bereichen des Gewindes (5) versehen ist mit einer bioaktiven Oberflächenbeschichtung (7), die durch Druck und Reibungswärme beim Eindrehen der Knochenschraube in Knochensubstanz zerstörbare Mikrokapseln (7b) mit einem Schmierstoff (7a) und/oder Mikro-Schleifkörper enthält.

6. Knochenschraube nach Anspruch 5, wobei die Mikro-Schleifkörper (7a) der Oberflächenbeschichtung (7) durch Druck und Reibungswärme beim Eindrehen der Knochenschraube in Knochensubstanz zerstörbar, insbesondere in kleinere Partikel umwandelbar sind, die eine Vergrößerung der effektiven Schraubenoberfläche bewirken.

7. Knochenschraube nach Anspruch 5 oder 6, wobei die Mikro-Schleifkörper (7a) kristallines Hydroxylapatit aufweisen und insbesondere nadelförmig ausgebildet sind.

8. Orthopädisches Implantat, das eine Knochenschraube (1) nach einem der vorangehenden Ansprüche oder einen entsprechenden angeformten Knochenschraubenabschnitt aufweist.

9. Verfahren zur Herstellung einer Knochenschraube (1) nach einem der Ansprüche 1 bis 7 oder eines orthopädischen Implantats nach Anspruch 8 mit den Schritten:
- Bereitstellen eines Grundkörpers (1'; 1") aus einer Magnesiumlegierung und
- Einformen des selbstschneidenden Gewindes (5) in die Außenwandung des Grundkörpers zur Herstellung der Knochenschraube oder des Knochenschraubenabschnitts des orthopädischen Implantats,
wobei im Schritt des Bereitstellens des Grundkörpers oder im Schritt des Einformens mindestens der Grundkontur des selbstschneidenden Gewindes durch eine drehende Führung eines Halbzeugs oder des Grundkörpers in einem Formwerkzeug (9) die der Eindrehrichtung des Gewindes entgegengerichtete Torsionseigenspannung eingeprägt wird.

10. Verfahren nach Anspruch 9, wobei der Grundkörper (1'; 1") als Rohr oder einen Rohrabschnitt aufweisendes Element bereitgestellt wird.

11. Verfahren nach Anspruch 9, wobei das Bereitstellen des Rohres (1'; 1") mit gleichzeitigem Einformen des selbstschneidenden Gewindes (5) in ein rotierendes Strangpressen, optional mit nachfolgendem Fräsen oder Schleifen oder Gewindeschneiden, erfolgt.

12. Verfahren nach Anspruch 11, wobei das rotierende Strangpressen als Warmumformen mit ein Verformungstemperatur im Bereich zwischen 100 °C und 450 °C und/oder mit einer Anzahl von 1,0 bis 8 Umdrehungen pro cm Schraubenlänge bei einem zwischen 2,0 und 3,5 mm liegenden Außendurchmesser ausgeführt wird.

13. Verfahren nach Anspruch 11 oder 12, wobei die Dehnrate zwischen 0,05 s⁻¹ und 25 s⁻¹, insbesondere zwischen 0,07 s⁻¹ und 22 s⁻¹, liegt.

14. Verfahren nach einem der Ansprüche 11 - 13, wobei der Materialabtrag beim nachfolgenden Fräsen oder Schleifen oder Gewindeschneiden weniger als 0,2 mm, insbesondere weniger als 0,1 mm, beträgt.

15. Verfahren nach einem der Ansprüche 9 - 14, weiter aufweisend die Schritte
- Ausbilden einer Beschichtungs-Basis (7) auf mindestens einem Abschnitt der Oberfläche des Gewindes, und
- Auf /Einbringen der Mikrokapseln (7b) und/oder der Mikro-Schleifkörper (7a) auf und/oder in die Beschichtungs-Basis.

## Claims

1. A bone screw (1) with self-tapping thread (5), consisting substantially of a magnesium alloy and provided with an impressed inherent torsional rigidity, which is directed oppositely to the screw-in direction of the thread.

2. The bone screw according to claim 1, wherein the impressed inherent torsional stress is constant over the screw length.

3. The bone screw according to one of the preceding claims, which has a cannulated (3) main body, in particular with an outer diameter in the range between 1.5 and 5 mm, especially between 2.5 and 3.5 mm, and an inner diameter in the range between 0.5 and 2.5 mm, especially between 0.8 and 1.3 mm.

4. The bone screw according to one of the preceding claims, wherein the self-tapping thread (5) has a saw-like fine-thread profile.

5. The bone screw according to one of the preceding claims, which is provided at least in regions of the thread (5) with a bioactive surface coating (7), which contains microcapsules (7b) with a lubricant (7a) and/or micro-abrasives, which microcapsules can be destroyed by pressure and frictional heat as the bone screw is screwed into bone substance.

6. The bone screw according to claim 5, wherein the micro-abrasives (7a) of the surface coating (7) can be destroyed, in particular can be converted into smaller particles, by pressure and frictional heat as the bone screw is screwed into bone substance, which particles enlarge the effective screw surface.

7. The bone screw according to claim 5 or 6, wherein the micro-abrasives (7a) comprise crystalline hydroxyapatite and in particular are needle-shaped.

8. An orthopedic implant, which comprises a bone screw (1) according to one of the preceding claims or an accordingly integrally formed bone screw portion.

9. A method for producing a bone screw (1) according to one of claims 1 to 7 or an orthopedic implant according to claim 8, comprising the steps of:
- providing a tube or a main body (1'; 1") from a magnesium alloy, and
- shaping the self-tapping thread (5) in the outer wall of the main body in order to produce the bone screw or the bone screw portion of the orthopedic implant,
wherein, during the step of providing the main body or during the step of shaping at least the basic contour of the self-tapping thread by guiding a semi-finished product or the main body in a die (9) in a rotary manner, the inherent torsional stress directed oppositely to the screw-in direction of the thread is impressed.

10. The method according to claim 9, wherein the main body (1'; 1") is provided as a tube or an element comprising a tube portion.

11. The method according to claim 9, wherein the tube (1'; 1") is provided with simultaneous shaping of the self-tapping thread (5) in a rotary extrusion process, optionally with subsequent milling or grinding or thread cutting.

12. The method according to claim 11, wherein the rotary extrusion is performed as hot working with a forming temperature in the range between 100 °C and 450 °C and/or with a number of 1.0 to 8 revolutions per cm of screw length with an outer diameter between 2.0 and 3.5 mm.

13. The method according to claim 11 or 12, wherein the strain rate is between 0.05 s⁻¹ and 25 s⁻¹, in particular between 0.07 s⁻¹ and 22 s⁻¹.

14. The method according to one of claims 11-13, wherein the material removal during subsequent milling or grinding or thread cutting is less than 0.2 mm, in particular less than 0.1 mm.

15. The method according to one of claims 9-14, further comprising the steps of
- forming a coating base (7) on at least one portion of the surface of the thread, and
- applying/introducing the microcapsules (7b) and/or the micro-abrasives (7a) to and/or into the coating base.

## Revendications

1. Vis pour os (1) avec un filetage (5) auto-taraudeur, constituée essentiellement d'un alliage de magnésium et pourvue d'une tension interne de torsion imposée qui est opposée à la direction de vissage du filetage.

2. Vis pour os selon la revendication 1, dans laquelle la tension interne de torsion imposée apporte une contribution constante sur la longueur de vis.

3. Vis pour os selon l'une des revendications précédentes, laquelle présente un corps de base (3) canulé, notamment avec un diamètre extérieur dans la plage entre 1,5 et 5 mm, plus spécialement entre 2,5 et 3,5 mm, et un diamètre intérieur dans la plage entre 0,5 et 2,5 mm, plus spécialement entre 0,8 et 1,3 mm.

4. Vis pour os selon l'une des revendications précédentes, dans laquelle le filetage (5) auto-taraudeur présente un profil de filetage en fines dents de scie.

5. Vis pour os selon l'une des revendications précédentes, laquelle est pourvue, au moins dans des zones du filetage (5), d'un revêtement de surface (7) bioactif qui contient des microcapsules (7b) avec un lubrifiant (7a), et/ou des micro corps de polissage, pouvant être détruits par la pression et la chaleur de frottement lors du vissage de la vis pour os dans la substance osseuse.

6. Vis pour os selon la revendication 5, dans laquelle les micro corps de polissage (7a) du revêtement de surface (7) peuvent être détruits, notamment transformés en plus petites particules, par la pression et la chaleur de frottement lors du vissage de la vis pour os dans la substance osseuse, qui provoquent une augmentation de la surface de vissage efficace.

7. Vis pour os selon la revendication 5 ou 6, dans laquelle les micro corps de polissage (7a) présentent de l'hydroxyapatite cristalline et sont notamment conçus sous la forme d'aiguilles.

8. Implant orthopédique qui présente une vis pour os (1) selon l'une des revendications précédentes ou une partie de vis pour os incorporée de manière correspondante.

9. Procédé de fabrication d'une vis pour os (1) selon l'une des revendications 1 à 7 ou d'un implant orthopédique selon la revendication 8, avec les étapes :
- de mise à disposition d'un corps de base (1' ; 1") à base d'un alliage de magnésium et
- d'incorporation du filetage auto-taraudeur (5) dans la paroi extérieure du corps de base pour la fabrication de la vis pour os ou de la partie de vis pour os de l'implant orthopédique,
où, dans l'étape de mise à disposition du corps de base ou dans l'étape de la mise en forme au moins du contour de base du filetage auto-taraudeur par un guidage en rotation d'un semi produit ou du corps de base dans un outil de mise en forme (9), la tension interne de torsion orientée à l'opposé du sens de vissage du filetage sera imposée.

10. Procédé selon la revendication 9, dans lequel le corps de base (1', 1") est mis à disposition sous la forme d'un élément présentant un tube ou une section de tube.

11. Procédé selon la revendication 9, dans lequel la mise à disposition du tube (1', 1") a lieu avec une incorporation simultanée du filetage (5) auto-taraudeur dans une extrusion par rotation, éventuellement avec un fraisage ultérieur ou un polissage, ou l'exécution d'un filetage.

12. Procédé selon la revendication 11, dans lequel l'extrusion par rotation est conçue sous la forme d'une transformation à chaud avec une température de transformation dans la plage entre 100 °C et 450 °C et/ou avec un nombre de 1,0 à 8 rotations par cm de longueur de vis pour un diamètre extérieur se situant entre 2,0 et 3,5 mm.

13. Procédé selon la revendication 11 ou 12, dans lequel le taux d'étirement se situe entre 0,05 s⁻¹et 25 s⁻¹, notamment entre 0,07 s⁻¹ et 22 s⁻¹

14. Procédé selon l'une des revendications 11 à 13, dans lequel le retrait de matière lors du fraisage ultérieur ou du polissage, ou de l'exécution du filetage, s'élève à moins de 0,2 mm, notamment à moins de 0,1 mm.

15. Procédé selon l'une des revendications 9 à 14, présentant en outre les étapes
- de formation d'une base de revêtement (7) sur au moins une section de la surface du filetage, et
- d'apport /d'incorporation des microcapsules (7b) et/ou des micro corps de polissage (7a) sur et/ou dans la base de revêtement.
